(19) Europäisches Patentamt
European Patent Office
Office européen des brevets

(11) EP 3 753 487 A1

(12) EUROPEAN PATENT APPLICATION

(43) Date of publication:
23.12.2020 Bulletin 2020/52

(51) Int Cl.:
A61B 5/042 (2006.01)
A61B 5/00 (2006.01)
A61B 5/06 (2006.01)
A61B 5/053 (2006.01)

(21) Application number: 20180659.3

(22) Date of filing: 18.06.2020

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 19.06.2019 US 201916446335

(71) Applicant: Biosense Webster (Israel) Ltd
Yokneam, 2066717 (IL)

(72) Inventors:
• GLINER, Vadim
2066717 Yokneam (IL)
• GOVARI, Assaf
2066717 Yokneam (IL)

(74) Representative: Small, Gary James
Carpmaels & Ransford LLP
One Southampton Row
London WC1B 5HA (GB)

(54) **MULTI-ARM PROBE RENDERING**

(57) In one embodiment, a medical procedure system, a probe including a shaft, deflectable arms, a position sensor on the shaft, and electrodes along each arm, and processing circuitry to measure readings of the sensor, compute first position coordinates of proximal ends of the arms responsively to the readings and a predefined spatial relation between the sensor and proximal ends, measure an indication of electrical impedances between body surface electrodes and at least two electrodes of each arm, compute second position coordinates of each of the at least two electrodes on each arm responsively to the indication, fit a respective curve corresponding to each arm responsively to the respective first position coordinates of the respective proximal end and the second position coordinates of each of the respective at least two electrodes, and render a graphical representation of the probe including the deflectable arms responsively to the respective fitted curve.

FIG. 1

EP 3 753 487 A1

## Description

FIELD OF THE INVENTION

The present invention relates to medical devices, and in particular, but not exclusively, to multi-arm probes.

BACKGROUND

Tracking the position of intrabody probes, such as insertion tubes, catheters and implants, is required for many medical procedures. A representation of the tracked probe may be displayed over a previously acquired body-part scan such as a CT, which is registered with a position tracking system tracking the probe, thereby allowing a physician to successfully navigate the probe in the body-part. For example, US Patent Publication 2017/0332971 of Macneil, et al., describes a system to generate a representation of a rhythm disorder that includes identifying remote or polar sources associated with a cardiac rhythm disorder. The system includes generating a representation based on cardiac information signals received from sensors by transformation of spline-sensor locations of a catheter to x-y coordinate pairs of locations. A first offset is determined resulting from a perturbation to corresponding x-y coordinate pairs of locations associated with the representation, the first offset displacing coordinate pairs of sensor locations of the representation by at least one unit of displacement in a first direction. A remote source associated with a cardiac rhythm disorder is identified when activations associated with the cardiac information signals rotate in sequence at least once, or emanate centrifugally for at least a first time period, the source being identified based on the representation as displaced.

US Patent Publication 2018/0303361 of Wu, et al., describes a catheter adapted for high density mapping and/or ablation of a tissue surface and has a distal electrode array with offset spine loops, each spine loop having at least a pair of linear portions and a distal portion connecting the pair of linear portions, and one or more electrodes on each linear portion. The linear portions of the plurality of offset spine loops are arranged in-plane of a single common plane, and the distal portions of the plurality of offset spine loops are arranged off-plane from the single common plane.

US Patent 6,574,492 to Ben-Haim, et al., describes a catheter for measuring physiological signals in a heart and comprises a structure at a distal end of the catheter wherein the structure has a plurality of arms, an electrode fixed to each arm and a device for generating position information located on each arm. The arms are located near the long axis of the catheter during insertion of the catheter within a heart and the arms are spreadable apart and away from the long axis of the catheter when the structure is within the heart.

US Patent Publication 2015/0351652 of Marecki, et al., describes an expandable electrode assembly for use in a cardiac mapping procedure and includes multiple bipolar electrode pairs including a first electrode located on an outer surface and a second electrode located on an inner surface of the individual splines forming the expandable electrode assembly. Such an electrode arrangement may produce improved electrical activation signals which may be used to produce a more accurate map of the electrical activity of a patient's heart.

SUMMARY

There is provided in accordance with an embodiment of the present disclosure, a medical procedure system, including a plurality of body surface electrodes configured to be applied to a skin surface of a living subject, a probe configured for inserting into a body-part of the living subject and including a shaft, a plurality of deflectable arms having respective proximal ends connected to a distal end of the shaft, a position sensor disposed on the shaft in a predefined spatial relation to the proximal ends of the deflectable arms, and multiple electrodes disposed at different, respective locations along each of the deflectable arms, and processing circuitry configured to measure electrical readings of the position sensor, compute first position coordinates of the proximal ends of the deflectable arms responsively to the measured electrical readings and the predefined spatial relation between the position sensor and the proximal ends of the deflectable arms, measure an indication of electrical impedances between the body surface electrodes and at least two of the multiple electrodes of each of the deflectable arms, compute second position coordinates of each of the at least two electrodes on each of the deflectable arms responsively to the indication of the electrical impedances, fit a respective curve corresponding to each of the deflectable arms responsively to the respective first position coordinates of the proximal end of the respective deflectable arm and the second position coordinates of each of the respective at least two electrodes, and render to the display a graphical representation of the probe including a graphical representation of the shaft, and a graphical representation of the deflectable arms responsively to the respective fitted curve.

Further in accordance with an embodiment of the present disclosure the processing circuitry is configured to compute third position coordinates of at least some of the multiple electrodes of each of the deflectable arms responsively to the respective fitted curve and the respective locations of the at least some multiple electrodes along the respective deflectable arm, and render to the display a graphical representation of the probe including a graphical representation of the shaft, and a graphical representation of the deflectable arms and the multiple electrodes responsively to the computed third position coordinates.

Still further in accordance with an embodiment of the present disclosure, the system includes a probe connector configured to couple the position sensor, and the at least two electrodes of each of the deflectable arms to the processing circuitry.

Additionally in accordance with an embodiment of the present disclosure the position sensor includes at least one shaft electrode, the processing circuitry being configured to measure another indication of electrical impedances between the body surface electrodes and the at least one shaft electrode, and compute the first position coordinates of the proximal ends of the deflectable arms responsively to the other indication of electrical impedances and the predefined spatial relation between the position sensor and the proximal ends of the deflectable arms.

Moreover, in accordance with an embodiment of the present disclosure at least two electrodes of each of the deflectable arms include only some of the multiple electrodes of each of the deflectable arms.

Further in accordance with an embodiment of the present disclosure the at least two electrodes of respective ones of the deflectable arms include a respective one of the multiple electrodes disposed furthest from the shaft.

Still further in accordance with an embodiment of the present disclosure the at least two electrodes include one of the multiple electrodes closest to a weakest part of the respective one of the deflectable arms, and the at least two electrodes of each of the deflectable arms include only some of the multiple electrodes of each of the deflectable arms

Additionally in accordance with an embodiment of the present disclosure the position sensor includes a magnetic sensor and at least one shaft electrode, the system further including a least one magnetic field radiator configured to transmit alternating magnetic fields into a region where the body-part is located, the magnetic sensor being configured to detect at least part of the transmitted alternating magnetic fields, the processing circuitry being configured to create a mapping between indications of electrical impedance and positions in a magnetic coordinate frame of the at least one magnetic field radiator.

Moreover, in accordance with an embodiment of the present disclosure the processing circuitry is configured to fit the curve for each of the deflectable arms based on a Bezier curve.

Further in accordance with an embodiment of the present disclosure the at least two electrodes of respective ones of the deflectable arms include a respective one of the multiple electrodes disposed furthest from the shaft, and the at least two electrodes include one of the multiple electrodes closest to a weakest part of the respective one of the deflectable arms.

There is also provided in accordance with another embodiment of the present disclosure, a medical procedure method, including applying a plurality of body surface electrodes to a skin surface of a living subject, inserting a probe into a body-part of the living subject, the probe including a shaft, a plurality of deflectable arms having respective proximal ends connected to a distal end of the shaft, a position sensor disposed on the shaft in a predefined spatial relation to the proximal ends of the deflectable arms, and multiple electrodes disposed at different, respective locations along each of the deflectable arms, measuring electrical readings of the position sensor, computing first position coordinates of the proximal ends of the deflectable arms responsively to the measured electrical readings and the predefined spatial relation between the position sensor and the proximal ends of the deflectable arms, measuring an indication of electrical impedances between the body surface electrodes and at least two of the multiple electrodes of each of the deflectable arms, computing second position coordinates of each of the at least two electrodes on each of the deflectable arms responsively to the indication of the electrical impedances, fitting a respective curve corresponding to each of the deflectable arms responsively to the respective first position coordinates of the proximal end of the respective deflectable arm and the second position coordinates of each of the respective at least two electrodes, and rendering to a display a graphical representation of the probe including a graphical representation of the shaft, and a graphical representation of the deflectable arms responsively to the respective fitted curve.

Still further in accordance with an embodiment of the present disclosure, the method includes computing third position coordinates of at least some of the multiple electrodes of each of the deflectable arms responsively to the respective fitted curve and the respective locations of the at least some multiple electrodes along the respective deflectable arm, and wherein the rendering including rendering to the display a graphical representation of the probe including a graphical representation of the shaft, and a graphical representation of the deflectable arms and the multiple electrodes responsively to the computed third position coordinates.

Additionally in accordance with an embodiment of the present disclosure the position sensor includes at least one shaft electrode, the method further including measuring another indication of electrical impedances between the body surface electrodes and the at least one shaft electrode, and computing the first position coordinates of the proximal ends of the deflectable arms responsively to the other indication of electrical impedances and the predefined spatial relation between the position sensor and the proximal ends of the deflectable arms.

Moreover, in accordance with an embodiment of the present disclosure at least two electrodes of each of the deflectable arms include only some of the multiple electrodes of each of the deflectable arms.

Further in accordance with an embodiment of the present disclosure the at least two electrodes of respective ones of the deflectable arms include a respective one of the multiple electrodes disposed furthest from the shaft.

Still further in accordance with an embodiment of the present disclosure the at least two electrodes include one of the multiple electrodes closest to a weakest part of the respective one of the deflectable arms, and the at least two electrodes of each of the deflectable arms include only some of the multiple electrodes of each of the deflectable arms

Additionally in accordance with an embodiment of the present disclosure the position sensor includes a magnetic sensor and at least one shaft electrode, the method further including transmitting alternating magnetic fields into a region where the body-part is located, detecting at least part of the transmitted alternating magnetic fields by the magnetic sensor, and creating a mapping between indications of electrical impedance and positions in a magnetic coordinate frame.

Moreover, in accordance with an embodiment of the present disclosure the fitting includes fitting the curve for each of the deflectable arms based on a Bezier curve.

Further in accordance with an embodiment of the present disclosure the at least two electrodes of respective ones of the deflectable arms include a respective one of the multiple electrodes disposed furthest from the shaft, and the at least two electrodes include one of the multiple electrodes closest to a weakest part of the respective one of the deflectable arms.

BRIEF DESCRIPTION OF THE DRAWINGS

The present invention will be understood from the following detailed description, taken in conjunction with the drawings in which:

Fig. 1 is a schematic view of a medical procedure system constructed and operative in accordance with an embodiment of the present invention;

Fig. 2 is a schematic view of a probe for use in the system of Fig. 1;

Fig. 3 is a schematic view of one of the deflectable arms of the probe of Fig. 2;

Fig. 4 is a schematic view of the arm of Fig. 3 illustrating computation of position coordinates of a proximal end of the deflectable arm of Fig. 3;

Fig. 5 is a schematic view of the arm of Fig. 3 illustrating fitting a curve based on the computed position coordinates of the proximal end of the deflectable arm and computed position coordinates of two electrodes of the deflectable arm;

Fig. 6 is a schematic view illustrating computed electrode positions along the fitted curve of Fig. 5;

Fig. 7 is a schematic view of a graphical representation of part of the probe of Fig. 2 rendered as a graphical overlay into an image of the heart according to the computed electrode positions of Fig. 6; and

Fig. 8 is a flowchart including exemplary steps in a method for use in the system of Fig. 1.

DESCRIPTION OF EXAMPLE EMBODIMENTS

OVERVIEW

As mentioned above, tracking the position of intrabody probes, such as insertion tubes, catheters and implants, is required for many medical procedures. A representation of a tracked probe may be displayed over a previously acquired body-part scan such as a CT image, which is registered with a position tracking system tracking the probe, thereby allowing a physician to successfully navigate the probe in the body-part.

Accurately rendering the tracked probe to a display may be difficult due to various reasons. For example, if the probe is flexible or deflectable, computing the location of the various parts of the probe may be limited by the number and location of position sensors on the probe.

In some systems, the number of position sensors which may be connected to a position tracking system may be limited by the capabilities of the position tracking system. For example, if the probe includes fifty electrodes for position tracking, but the position tracking system has a smaller number of input terminals, such as twenty terminals, for connection to the electrodes, computing the locations of only twenty of the electrodes may not provide an accurate enough picture of the various parts of the probe. An example of a fifty-electrode probe is the Octaray® catheter produced by Biosense Webster Inc., of Irvine, CA, USA. The Octaray includes eight deflectable arms disposed at the distal end of a shaft, with each of the deflectable arms including six electrodes and another two electrodes disposed on the shaft on either side of a magnetic sensor. An example of a five-arm probe is the Pentaray® catheter of Biosense Webster Inc.

Embodiments of the invention provide a system and method that address these problems, in order to accurately estimate the disposition of a probe having multiple deflectable arms within the body of a living subject. These embodiments take advantage of prior knowledge of the geometrical and mechanical properties of the probe, and particularly of its arms, in order to produce an accurate graphical representation of the probe while using only a small number of position sensors along each of the arms. Thus, the present embodiments improve the reliability of image display while reducing the data input requirements of the system.

In the disclosed embodiments, each of the arms has a proximal end connected to a shaft of the probe and a distal end furthest from the shaft. The distal end has free movement subject to the flexibility of the arm and the various obstacles encountered in the body of the living subject. The arms include electrodes disposed at different, respective locations along each deflectable arm. The probe may include any suitable number of deflectable arms, for example, but not limited to, five or eight. Each of the arms may include any suitable number of electrodes, for example, but not limited to, two or more electrodes.

The disposition of each arm is estimated based on fitting a curve to computed position coordinates of at least three points along the respective arm. As will be explained in more detail below, the disposition of each of the arms may be estimated based on position data provided by a minimal number of arm electrodes, which is particularly advantageous when the position tracking system has a smaller number of input terminals for connection to the electrodes.

The position coordinates of one of the points (first point) used in the curve fitting may be based on computed position coordinates of an electrode disposed close to the distal tip of the respective arm.

The position coordinates of another one of the points (second point) used in the curve fitting may be based on computed position coordinates of the proximal end of the respective arm. In some embodiments, the position coordinates of the proximal end of the respective arm may be computed based on position sensing of a position sensor disposed on the shaft and a predefined spatial relation between the shaft position sensor and the proximal end of the respective arm.

The position coordinates of yet another point (third point) used in the curve fitting may be based on the computed position coordinates of an electrode disposed on the respective arm between the electrode close to the distal tip and the proximal end of the respective arm. In some embodiments, the computed position coordinates of the third point are used in the curve fitting may be selected based on the electrode closest to the weakest part of each arm. The weakest part of the arm is typically the part of the arm that bends through the largest angle and/or with the smallest bend radius, and therefore provides particularly relevant data for use in fitting the curves to estimate the dispositions of the deflectable arms.

Processing circuitry measures electrical readings of the position sensor disposed on the shaft and computes first position coordinates of the proximal ends of the deflectable arms responsively to the measured electrical readings and the predefined spatial relation between the position sensor and the proximal ends of the deflectable arms.

For the purposes of tracking the electrode locations on the probe, body surface electrodes are applied to the skin surface of the living subject. The processing circuitry measures an indication of electrical impedances between the body surface electrodes and the arm electrodes used to provide position coordinates used in the curve fitting. In some embodiments, the arm electrodes used in the curve fitting may comprise only some of the multiple electrodes of each of the deflectable arms. In other embodiment, the arm electrodes used in the curve fitting may comprise all of the electrodes of each of the deflectable arms.

The processing circuitry computes second position coordinates of each of the electrodes used in the curve fitting responsively to the indication of the electrical impedances.

The processing circuitry fits a respective curve corresponding to each of the deflectable arms responsively to the respective first position coordinates of the proximal end of the respective arm and the second position coordinates of each of the respective electrodes. Fitting the curves may be based on a Bezier curve or any other suitable curve fitting method.

The processing circuitry computes position coordinates of at least some, and generally all, of the electrodes of each of the deflectable arms responsively to the respective fitted curve and the respective locations of the electrodes (for which the computing is currently being performed) along the respective deflectable arm. The computation estimates where the electrodes would be positioned assuming that the electrodes are positioned along the respective fitted curve and the electrodes are located on the respective fitted curve according to the known locations of the electrodes along each of the arms.

The processing circuitry renders to a display a graphical representation of the probe including a graphical representation of the shaft, and a graphical representation of the deflectable arms with the electrodes responsively to the computed positions of the electrodes. The graphical representation of each deflectable arm may be based on connecting the computed position coordinates of the electrodes of the respective deflectable arm, the computed position of the proximal end of the respective deflectable arm, and the end of the respective deflectable arm (computed from a known length of the arm from the proximal end) with straight lines or curves. Representations of the electrodes may be rendered on the deflectable arms according to the computed position coordinates of the electrodes.

SYSTEM DESCRIPTION

Documents incorporated by reference herein are to be considered an integral part of the application except that, to the extent that any terms are defined in these incorporated documents in a manner that conflicts with definitions made explicitly or implicitly in the present specification, only the definitions in the present specification should be considered.

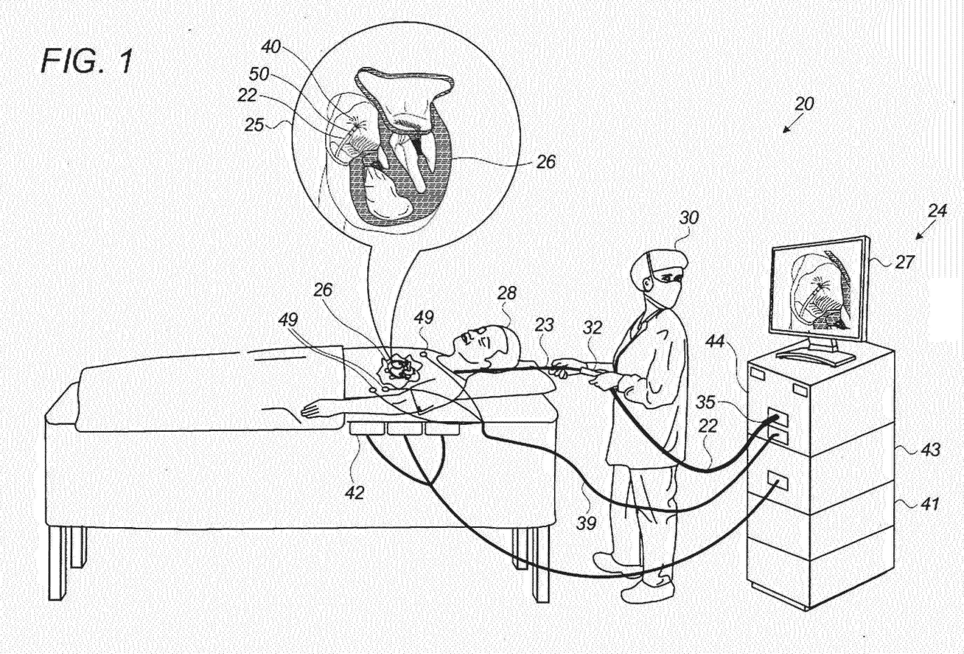

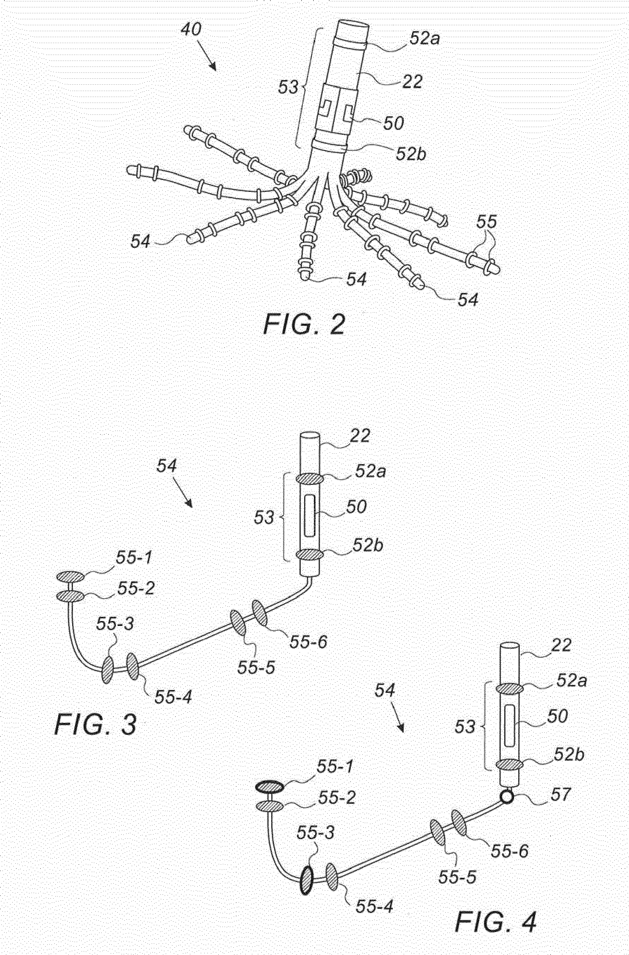

Reference is now made to Fig. 1, which is a schematic view of a medical procedure system 20 constructed and operative in accordance with an embodiment of the present invention. Reference is also made to Fig. 2, which is a schematic view of a probe 40 for use in the system 20 of Fig. 1.

The medical procedure system 20 is used to determine the position of the probe 40, seen in an inset 25 of Fig. 1 and in more detail in Fig. 2. The probe 40 includes a shaft 22 and a plurality of deflectable arms 54 (only some labeled for the sake of simplicity) for inserting into a body-part of a living subject. The deflectable arms 54 have respective proximal ends connected to the distal end of the shaft 22.

The probe 40 includes a position sensor 53 disposed on the shaft 22 in a predefined spatial relation to the proximal ends of the deflectable arms 54. The position sensor 53 may include a magnetic sensor 50 and/or at least one shaft electrode 52. The magnetic sensor 50 may include at least one coil, for example, but not limited to, a dual-axis or a triple axis coil arrangement to provide position data for location and orientation including roll. The probe 40 includes multiple electrodes 55 (only some are labeled in Fig. 2 for the sake of simplicity) disposed at different, respective locations along each of the deflectable arms 54. Typically, the probe 40 may be used for mapping electrical activity in a heart of the living subject using the electrodes 55, or for performing any other suitable function in a body-part of a living subject.

The medical procedure system 20 may determine a position and orientation of the shaft 22 of the probe 40 based on signals provided by the magnetic sensor 50 and/or the shaft electrodes 52 (proximal-electrode 52a and distal-electrode 52b) fitted on the shaft 22, on either side of the magnetic sensor 50. The proximal-electrode 52a, the distal-electrode 52b, the magnetic sensor 50 and at least some of the electrodes 55 are connected by wires running through the shaft 22 via a probe connector 35 to various driver circuitries in a console 24. In some embodiments, at least two of the electrodes 55 of each of the deflectable arms 54, the shaft electrodes 52, and the magnetic sensor 50 are connected to the driver circuitries in the console 24 via the probe connector 35. In some embodiments, the distal-electrode 52b and/or the proximal electrode 52a may be omitted.

The illustration shown in Fig. 2 is chosen purely for the sake of conceptual clarity. Other configurations of shaft electrodes 52 and electrodes 55 are possible. Additional functionalities may be included in the position sensor 53. Elements which are not relevant to the disclosed embodiments of the invention, such as irrigation ports, are omitted for the sake of clarity.

A physician 30 navigates the probe 40 to a target location in a body part (e.g., a heart 26) of a patient 28 by manipulating the shaft 22 using a manipulator 32 near the proximal end of the probe 40 and/or deflection from a sheath 23. The probe 40 is inserted through the sheath 23, with the deflectable arms 54 gathered together, and only after the probe 40 is retracted from the sheath 23, the deflectable arms 54 are able to spread and regain their intended functional shape. By containing deflectable arms 54 together, the sheath 23 also serves to minimize vascular trauma on its way to the target location.

Console 24 comprises processing circuitry 41, typically a general-purpose computer and a suitable front end and interface circuits 44 for generating signals in, and/or receiving signals from, body surface electrodes 49 which are attached by wires running through a cable 39 to the chest and to the back, or any other suitable skin surface, of the patient 28.

Console 24 further comprises a magnetic-sensing sub-system. The patient 28 is placed in a magnetic field generated by a pad containing at least one magnetic field radiator 42, which is driven by a unit 43 disposed in the console 24. The magnetic field radiator(s) 42 is configured to transmit alternating magnetic fields into a region where the body-part (e.g., the heart 26) is located. The magnetic fields generated by the magnetic field radiator(s) 42 generate direction signals in the magnetic sensor 50. The magnetic sensor 50 is configured to detect at least part of the transmitted alternating magnetic fields and provide the direction signals as corresponding electrical inputs to the processing circuitry 41.

In some embodiments, the processing circuitry 41 uses the position-signals received from the shaft electrodes 52, the magnetic sensor 50 and the electrodes 55 to estimate a position of the probe 40 inside an organ, such as inside a cardiac chamber. In some embodiments, the processing circuitry 41 correlates the position signals received from the electrodes 52, 55 with previously acquired magnetic location-calibrated position signals, to estimate the position of the probe 40 inside a cardiac chamber. The position coordinates of the shaft electrodes 52 and the electrodes 55 may be determined by the processing circuitry 41 based on, among other inputs, measured impedances, or on proportions of currents distribution, between the electrodes 52, 55 and the body surface electrodes 49. The console 24 drives a display 27, which shows the distal end of the probe 40 inside the heart 26.

The method of position sensing using current distribution measurements and/or external magnetic fields is implemented in various medical applications, for example, in the Carto® system, produced by Biosense Webster Inc. (Irvine, California), and is described in detail in U.S. Patents 5,391,199, 6,690,963, 6,484,118, 6,239,724, 6,618,612, 6,332,089, 7,756,576, 7,869,865, and 7,848,787, in PCT Patent Publication WO 96/05768, and in U.S. Patent Application Publications 2002/0065455 A1, 2003/0120150 A1 and 2004/0068178 A1, whose disclosures are all incorporated herein by reference.

The Carto®3 system applies an Active Current Location (ACL) impedance-based position-tracking method. In some embodiments, using the ACL method, the processing circuitry 41 is configured to create a mapping (e.g., current-position matrix (CPM)) between indications of electrical impedance and positions in a magnetic coordinate frame of the magnetic field radiator(s) 42. The processing circuitry 41 estimates the positions of the shaft electrodes 52 and the electrodes 55 by performing a lookup in the CPM.

Processing circuitry 41 is typically programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

Fig. 1 shows only elements related to the disclosed techniques, for the sake of simplicity and clarity. The system 20 typically comprises additional modules and elements that are not directly related to the disclosed techniques, and thus are intentionally omitted from Fig. 1 and from the corresponding description.

Reference is now made to Fig. 3, which is a schematic view of one of the deflectable arms 54 of the probe 40 of Fig. 2. Fig. 3 shows the electrodes 55 labeled individually from 55-1 to 55-6. The electrode 55-1 is closest to the distal end of the deflectable arm 54, while the electrode 55-6 is closest to the proximal end of the deflectable arm 54. The electrode 55-3 is closest to the weakest part of the deflectable arm 54 which in Fig. 3 is indicated by the deflectable arm 54 being the most curved near the electrode 55-3.

Reference is now made to Fig. 4, which is a schematic view of the arm 54 of Fig. 3 illustrating computation of position coordinates of a proximal end 57 of the deflectable arm 54 of Fig. 3. The proximal end 57 of the deflectable arm 54 may be computed responsively to: the predefined spatial relation between the position sensor 53 (e.g., the magnetic sensor 50 and/or the shaft electrodes 52) and the proximal ends 57 of the deflectable arms 54; and computed position coordinates of the magnetic sensor 50 and/or computed position coordinates of the shaft electrodes 52.

The electrodes 55-1 and 55-3 (which comprise a minority of the six electrodes 55 on the deflectable arm 54) have been selected in the example of Fig. 4 to be connected to the console 24 (Fig. 1) via the probe connector 35 (Fig. 1) to provide signals for use in computing the position coordinates of the electrodes 55-1 and 55-3. Therefore, for the eight deflectable arms 54 of the probe 40 there are sixteen connections from the electrodes 55 and two connections from the shaft electrode 52 to the console 24 via the probe connector 35. It should be noted that more other electrodes 55 may be connected to the console 24 or an auxiliary unit for measuring electrical activity of the body-part (e.g., the heart 26).

As mentioned above with reference to Fig. 3, the electrode 55-1 is the closest to the distal end of the deflectable arm 54 (and therefore furthest from the shaft 22) while the electrode 55-3 is closest to the weakest part of the deflectable arm 54. Therefore, the electrodes 55-1, 55-3 are suitable candidates for use in fitting a curve to determine the shape of the deflectable arm 54.

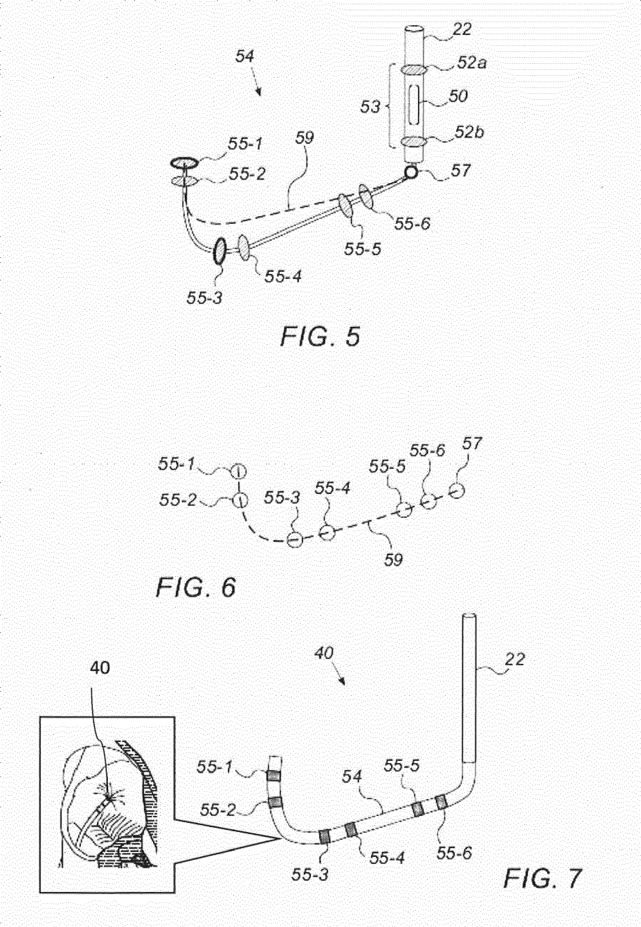

The computed position coordinates of the electrodes 55-1, 55-3 and the proximal end 57 provide data for fitting a curve, described in more detail with reference to Fig. 5.

Reference is now made to Fig. 5, which is a schematic view of the arm 54 of Fig. 3 illustrating fitting a curve 59 based on the computed position coordinates of the proximal end 57 of the deflectable arm 54 and computed position coordinates of two electrodes 55-1, 55-3 of the deflectable arm 54.

The fitting of the curve 59 may be performed using any suitable curve fitting method, for example, but not limited to, a Bezier curve. A Bezier curve is a smooth curve that is typically defined by two end points (e.g., the position coordinates of the electrode 55-1 and the proximal end 57) and at least one midpoint (e.g., the position coordinates of the electrode 55-3). The Bezier method defines a curve which goes through the two end-points and is influenced (e.g., pulled) by the mid-point(s). The Bezier curve may, or may not, go through the midpoint(s).

A quadratic Bezier curve may be used to provide curve fitting for three data points. A quadratic Bezier curve is the path traced by the function B(t) given points $P_0$, $P_1$ and $P_2$, where

$$B(t) = (1-t)[(1-t)P_0 + tP_1] + t[(1-t)P_1 + tP_2],$$

and the curve is generated by incrementing t from 0 to 1.

A cubic Bezier curve may be used to provide curve fitting for four data points. A cubic Bezier curve is the path traced by the function B(t) given points $P_0$, $P_1$, $P_2$, and $P_3$ where

$$B(t) = (1 - t)^3 P_0 + 3(1 - t)^2 t P_1 + 3(1 - t) t^2 P_2 + t^3 P_3,$$

and the curve is generated by incrementing t from 0 to 1.

Higher order Bezier curves may be used to provide curve fitting for more than four data points.

Reference is now made to Fig. 6, which is a schematic view illustrating computed electrode positions along the fitted curve 59 of Fig. 5.

Position coordinates of at least some, and generally all, of the multiple electrodes 55 of each of the deflectable arms 54 are computed responsively to the respective fitted curve 59 and the respective locations of the multiple electrodes 55 (for which the computing is currently being performed) along the respective deflectable arm 54. The computation estimates where the electrodes 55 would be positioned assuming that the electrodes 55 are positioned along the respective fitted curve 59. The computation step uses the respective fitted curve 59 and the known locations of the electrodes 55 along the respective deflectable arm 54 as input to compute (estimate) the positions of the electrodes 55 assuming the electrodes 55 are located on the respective fitted curve 59 according to the known locations of the electrodes 55 along the respective arm 54. For example, the known locations (spacing) of the electrodes 55 along the arms 54 may be used to compute the position coordinates of the electrodes 55 along the respective curve 59 either starting at the end of the respective curve 59 corresponding with the position of the electrode 55-1 or from the end of the respective curve 59 corresponding with the proximal end 57.

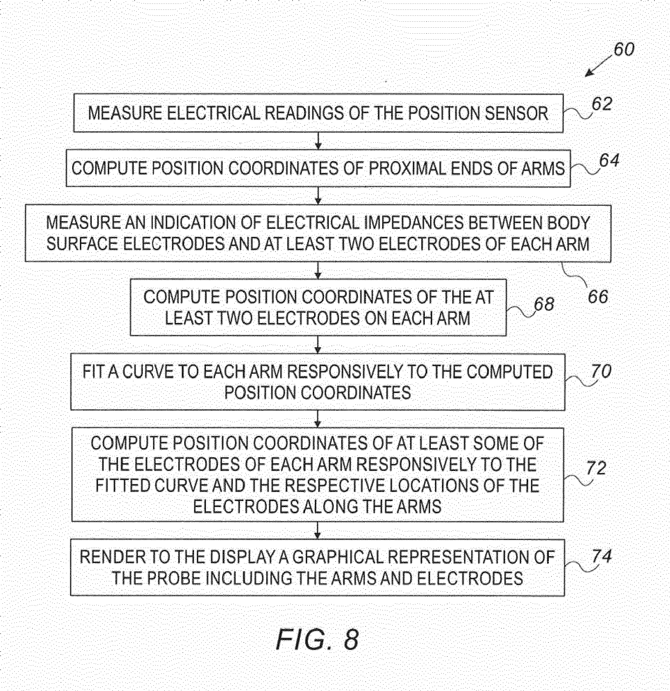

Reference is now made to Fig. 7, which is a schematic view of a graphical representation of part of the probe 40 of Fig. 2 on a display screen rendered according to the computed electrode positions of Fig. 6. The graphical representation of each deflectable arm 55 may be based on connecting the computed electrode positions of the respective arm 54, the computed position of the proximal end 57 of the respective arm 54, and the end of the respective arm 54 (computed from the known length of the respective arm 54 from its proximal end 57) with straight lines or curves. Representations of the electrodes 55 may be rendered on the deflectable arms 54 according to the computed position coordinates of the electrodes 55 (computed in the step of block 72). The shaft 22 may be computed based on the computed positions of the shaft electrode(s) 52 and/or the magnetic sensor 50. Reference is now made to Fig. 8, which is a flowchart 60 including exemplary steps in a method for use in the system 20 of Fig. 1. Reference is also made to Figs. 1 and 5.

The processing circuitry 41 is configured to measure (block 62) electrical readings of the position sensor 53. The processing circuitry 41 is configured to compute (block 64) position coordinates of the proximal ends 57 of the deflectable arms 54 responsively to the measured electrical readings and the predefined spatial relation between the position sensor 53 and the proximal ends 57 of the deflectable arms 54.

In some embodiments, the position coordinates of the proximal end 57 may be computed based on a computed position of the magnetic sensor 50.

In some embodiments, the processing circuitry 41 is configured to measure an indication of electrical impedances between the body surface electrodes 49 and the shaft electrode(s) 52 and compute the position coordinates of the proximal ends 57 of the deflectable arms 54 responsively to the measured indication of electrical impedances and the predefined spatial relation between the position sensor 53 (e.g., the shaft electrode(s) 52) and the proximal ends 57 of the deflectable arms 54.

The processing circuitry 41 is configured to measure (block 66) an indication of electrical impedances between the body surface electrodes 49 and at least two of the multiple electrodes 55 (e.g., the electrode 55-1, and the electrode 55-3) of each of the deflectable arms 54. The processing circuitry 41 is configured to compute (block 68) position coordinates of each of the at least two electrodes 55 on each of the deflectable arms 54 responsively to the indication of the electrical impedances measured in the step of block 66.

The processing circuitry 41 is configured to fit (block 70) a respective curve 59 corresponding to each deflectable arm 54 responsively to the respective position coordinates of the position sensor 53 and the position coordinates of each of the respective at least two electrodes 55. In some embodiments, the processing circuitry 41 is configured to fit the curve 59 for each of the deflectable arms 54 based on a Bezier curve. In other embodiments, the fitting of the curve 59 may be performed according to any suitable curve fitting method.

The processing circuitry 41 is configured to compute (block 72) position coordinates of at least some, and generally all, of the multiple electrodes 55 of each of the deflectable arms 54 responsively to the respective fitted curve 59 and the respective locations of the multiple electrodes 55 (for which the computing is currently being performed) along the respective deflectable arm 54. The computation step of block 72 estimates where the electrodes 55 would be positioned assuming that the electrodes 55 are positioned along the respective fitted curve 59. The computation step of block 72 uses the respective fitted curve 59 and the known locations of the electrodes 55 along the respective deflectable arm 54 as input to compute (estimate) the positions of the electrodes 55 assuming the electrodes 55 are located on the respective fitted curve 59 according to the known locations of the electrodes 55 along each of the arms 54. For example, the known locations (spacing) of the electrodes 55 along the arms 54 may be used to compute the position coordinates of the electrodes 55 along the respective curve 59 either starting at the end of the respective curve 59 corresponding with the position of the electrode 55-1 or from the end of the respective curve 59 corresponding with the proximal end 57.

The processing circuitry 41 is configured to render (block 74) to the display 27 a graphical representation of the probe 40 including a graphical representation of the shaft 22, and a graphical representation of the deflectable arms 54 including the electrode 55 responsively to the computed position coordinates of the electrodes 55 (which were computed responsively to the respective fitted curve 57. The graphical representation of each deflectable arm 54 may be based on connecting the computed position coordinates of the electrodes 55 (computed in the step of block 72) of the respective arm 54, the computed position of the proximal end 57 of the respective arm 54, and the end of the respective arm 54 (from the known length of the respective arm 54 from the proximal end 57) with straight lines or curves. Representations of the electrodes 55 may be rendered on the deflectable arms 54 according to the computed position coordinates of the electrodes 55 (computed in the step of block 72). The shaft 22 may be computed based on the computed positions of the shaft electrode(s) 52 and/or the magnetic sensor 50.

An example of a graphical representation of the probe 40 is shown in Fig. 2 and a portion of the probe 40 is shown in Fig. 7. The representation of the probe 40 and the relevant spine 54 with electrodes 55-1; 55-2; 55-3; 55-4; 55-5; and 55-6 of probe 40 may be displayed on display screen 27 (Fig. 1) over a previously acquired body-part scan such as a CT scan or MRI scan of the heart (or any other organ), which is registered and merged with a position tracking system (e.g., Carto 3 system by Biosense Ltd., Irvine, CA) tracking the probe 40, thereby allowing the physician 30 to successfully navigate the probe 40 in the body-part in real-time by observing the display screen 27. By extension of the technique described and illustrated here, all of the spines 54 and their respective electrodes for probe 40 can also be determined and rendered visually with all spines and electrodes (e.g., Fig. 2) on a display screen overlaying anatomical images previously acquired or generated by the Carto3 system.

Various features of the invention which are, for clarity, described in the contexts of separate embodiments may also be provided in combination in a single embodiment. Conversely, various features of the invention which are, for brevity, described in the context of a single embodiment may also be provided separately or in any suitable sub-combination.

The embodiments described above are cited by way of example, and the present invention is not limited by what has been particularly shown and described hereinabove. Rather the scope of the invention includes both combinations and subcombinations of the various features described hereinabove, as well as variations and modifications thereof which would occur to persons skilled in the art upon reading the foregoing description and which are not disclosed in the prior art.

ASPECTS OF THE INVENTION

1. A medical procedure method, comprising:

applying a plurality of body surface electrodes to a skin surface of a living subject;
inserting a probe into a body-part of the living subject, the probe comprising: a shaft; a plurality of deflectable arms having respective proximal ends connected to a distal end of the shaft; a position sensor disposed on the shaft in a predefined spatial relation to the proximal ends of the deflectable arms; and multiple electrodes disposed at different, respective locations along each of the deflectable arms;
measuring electrical readings of the position sensor;
computing first position coordinates of the proximal ends of the deflectable arms responsively to the measured electrical readings and the predefined spatial relation between the position sensor and the proximal ends of the deflectable arms;
measuring an indication of electrical impedances between the body surface electrodes and at least two of the multiple electrodes of each of the deflectable arms;
computing second position coordinates of each of the at least two electrodes on each of the deflectable arms responsively to the indication of the electrical impedances;
fitting a respective curve corresponding to each of the deflectable arms responsively to the respective first position coordinates of the proximal end of the respective deflectable arm and the second position coordinates of each of the respective at least two electrodes; and
rendering to a display a graphical representation of the probe including a graphical representation of the shaft, and a graphical representation of the deflectable arms responsively to the respective fitted curve.

2. The method according to aspect 1, further comprising computing third position coordinates of at least some of the multiple electrodes of each of the deflectable arms responsively to the respective fitted curve and the respective locations of the at least some multiple electrodes along the respective deflectable arm, and wherein the rendering including rendering to the display a graphical representation of the probe including a graphical representation of the shaft, and a graphical representation of the deflectable arms and the multiple electrodes responsively to the computed third position coordinates.

3. The method according to aspect 1, wherein the position sensor includes at least one shaft electrode, the method further comprising:
measuring another indication of electrical impedances between the body surface electrodes and the at least one shaft electrode; and
computing the first position coordinates of the proximal ends of the deflectable arms responsively to the other indication of electrical impedances and the predefined spatial relation between the position sensor and the proximal ends of the deflectable arms.

4. The method according to aspect 1, wherein at least two electrodes of each of the deflectable arms comprise only some of the multiple electrodes of each of the deflectable arms.

5. The method according to aspect 1, wherein the at least two electrodes of respective ones of the deflectable arms include a respective one of the multiple electrodes disposed furthest from the shaft.

6. The method according to aspect 5, wherein: the at least two electrodes include one of the multiple electrodes closest to a weakest part of the respective one of the deflectable arms; and the at least two electrodes of each of the deflectable arms comprise only some of the multiple electrodes of each of the deflectable arms.

7. The method according to aspect 6, wherein the position sensor comprises a magnetic sensor and at least one shaft electrode, the method further comprising: transmitting alternating magnetic fields into a region where the body-part is located; detecting at least part of the transmitted alternating magnetic fields by the magnetic sensor; and creating a mapping between indications of electrical impedance and positions in a magnetic coordinate frame.

8. The method according to aspect 1, wherein the fitting includes fitting the curve for each of the deflectable arms based on a Bezier curve.

9. The method according to aspect 8, wherein: the at least two electrodes of respective ones of the deflectable arms include a respective one of the multiple electrodes disposed furthest from the shaft; and the at least two electrodes include one of the multiple electrodes closest to a weakest part of the respective one of the deflectable arms.

**Claims**

1.  A medical procedure system, comprising:

    a plurality of body surface electrodes configured to be applied to a skin surface of a living subject;
    a probe configured for inserting into a body-part of the living subject and comprising: a shaft; a plurality of deflectable arms having respective proximal ends connected to a distal end of the shaft; a position sensor disposed on the shaft in a predefined spatial relation to the proximal ends of the deflectable arms; and multiple electrodes disposed at different, respective locations along each of the deflectable arms; and
    processing circuitry configured to:

       measure electrical readings of the position sensor;
       compute first position coordinates of the proximal ends of the deflectable arms responsively to the measured electrical readings and the predefined spatial relation between the position sensor and the proximal ends of the deflectable arms;
       measure an indication of electrical impedances between the body surface electrodes and at least two of the multiple electrodes of each of the deflectable arms;
       compute second position coordinates of each of the at least two electrodes on each of the deflectable arms responsively to the indication of the electrical impedances;
       fit a respective curve corresponding to each of the deflectable arms responsively to the respective first position coordinates of the proximal end of the respective deflectable arm and the second position coordinates of each of the respective at least two electrodes; and
render to the display a graphical representation of the probe including a graphical representation of the shaft, and a graphical representation of the deflectable arms responsively to the respective fitted curve.

2. The system according to claim 1, wherein the processing circuitry is configured to:

compute third position coordinates of at least some of the multiple electrodes of each of the deflectable arms responsively to the respective fitted curve and the respective locations of the at least some multiple electrodes along the respective deflectable arm; and
render to the display a graphical representation of the probe including a graphical representation of the shaft, and a graphical representation of the deflectable arms and the multiple electrodes responsively to the computed third position coordinates.

3. The system according to claim 1, further comprising a probe connector configured to couple the position sensor, and the at least two electrodes of each of the deflectable arms to the processing circuitry.

4. The system according to claim 1, wherein the position sensor includes at least one shaft electrode, the processing circuitry being configured to:

measure another indication of electrical impedances between the body surface electrodes and the at least one shaft electrode; and
compute the first position coordinates of the proximal ends of the deflectable arms responsively to the other indication of electrical impedances and the predefined spatial relation between the position sensor and the proximal ends of the deflectable arms.

5. The system according to claim 1, wherein at least two electrodes of each of the deflectable arms comprise only some of the multiple electrodes of each of the deflectable arms.

6. The system according to claim 1, wherein the at least two electrodes of respective ones of the deflectable arms include a respective one of the multiple electrodes disposed furthest from the shaft.

7. The system according to claim 6, wherein: the at least two electrodes include one of the multiple electrodes closest to a weakest part of the respective one of the deflectable arms; and the at least two electrodes of each of the deflectable arms comprise only some of the multiple electrodes of each of the deflectable arms.

8. The system according to claim 7, wherein the position sensor comprises a magnetic sensor and at least one shaft electrode; the system further comprising a least one magnetic field radiator configured to transmit alternating magnetic fields into a region where the body-part is located, the magnetic sensor being configured to detect at least part of the transmitted alternating magnetic fields; the processing circuitry being configured to create a mapping between indications of electrical impedance and positions in a magnetic coordinate frame of the at least one magnetic field radiator.

9. The system according to claim 1, wherein the processing circuitry is configured to fit the curve for each of the deflectable arms based on a Bezier curve.

10. The system according to claim 9, wherein: the at least two electrodes of respective ones of the deflectable arms include a respective one of the multiple electrodes disposed furthest from the shaft; and the at least two electrodes include one of the multiple electrodes closest to a weakest part of the respective one of the deflectable arms.

FIG. 1

*FIG. 2*

*FIG. 3*

*FIG. 4*

FIG. 5

FIG. 6

FIG. 7

MEASURE ELECTRICAL READINGS OF THE POSITION SENSOR — 62

COMPUTE POSITION COORDINATES OF PROXIMAL ENDS OF ARMS — 64

MEASURE AN INDICATION OF ELECTRICAL IMPEDANCES BETWEEN BODY SURFACE ELECTRODES AND AT LEAST TWO ELECTRODES OF EACH ARM — 66

COMPUTE POSITION COORDINATES OF THE AT LEAST TWO ELECTRODES ON EACH ARM — 68

FIT A CURVE TO EACH ARM RESPONSIVELY TO THE COMPUTED POSITION COORDINATES — 70

COMPUTE POSITION COORDINATES OF AT LEAST SOME OF THE ELECTRODES OF EACH ARM RESPONSIVELY TO THE FITTED CURVE AND THE RESPECTIVE LOCATIONS OF THE ELECTRODES ALONG THE ARMS — 72

RENDER TO THE DISPLAY A GRAPHICAL REPRESENTATION OF THE PROBE INCLUDING THE ARMS AND ELECTRODES — 74

60

FIG. 8

European Patent Office
Europäisches Patentamt
European Patent Office
Office européen des brevets

# EUROPEAN SEARCH REPORT

Application Number

EP 20 18 0659

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 3 181 046 A1 (BIOSENSE WEBSTER (ISRAEL) LTD [IL]) 21 June 2017 (2017-06-21) * figures 1-3,6 * * paragraph [0018] * * paragraph [0022] * * paragraph [0026] * * paragraph [0029] - paragraph [0035] * * paragraph [0038] * * paragraph [0048] * ----- | 1-10 | INV. A61B5/042 A61B5/06 A61B5/00 A61B5/053 |
| Y | CN 106 606 372 A (SICHUAN JINJIANG ELECTRONIC SCIENCE & TECH CO LTD) 3 May 2017 (2017-05-03) * paragraph [0013] - paragraph [0017] * * paragraph [0025] * * paragraph [0048] * ----- | 1-10 | |
| A | EP 3 449 821 A1 (BIOSENSE WEBSTER ISRAEL LTD [IL]) 6 March 2019 (2019-03-06) * figures 1,4 * * paragraph [0026] - paragraph [0031] * * paragraph [0044] - paragraph [0047] * ----- | 1 | |

| | | | TECHNICAL FIELDS SEARCHED (IPC) |
|---|---|---|---|
| | | | A61B |

The present search report has been drawn up for all claims

| Place of search | Date of completion of the search | Examiner |
|---|---|---|
| The Hague | 31 July 2020 | Oancea, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 18 0659

This annex lists the patent family members relating to the patent documents cited in the above-mentioned European search report.
The members are as contained in the European Patent Office EDP file on
The European Patent Office is in no way liable for these particulars which are merely given for the purpose of information.

31-07-2020

| Patent document cited in search report | | Publication date | Patent family member(s) | | Publication date |
|---|---|---|---|---|---|
| EP 3181046 | A1 | 21-06-2017 | AU | 2016259376 A1 | 06-07-2017 |
| | | | CA | 2950609 A1 | 18-06-2017 |
| | | | CN | 106923829 A | 07-07-2017 |
| | | | EP | 3181046 A1 | 21-06-2017 |
| | | | ES | 2736532 T3 | 02-01-2020 |
| | | | IL | 249198 A | 30-04-2020 |
| | | | JP | 2017109101 A | 22-06-2017 |
| | | | US | 2017172455 A1 | 22-06-2017 |
| | | | US | 2019343423 A1 | 14-11-2019 |
| CN 106606372 | A | 03-05-2017 | NONE | | |
| EP 3449821 | A1 | 06-03-2019 | AU | 2018211320 A1 | 07-03-2019 |
| | | | CA | 3012942 A1 | 21-02-2019 |
| | | | CN | 109419501 A | 05-03-2019 |
| | | | EP | 3449821 A1 | 06-03-2019 |
| | | | JP | 2019034152 A | 07-03-2019 |
| | | | US | 2019053708 A1 | 21-02-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20170332971 A, Macneil
- US 20180303361 A, Wu
- US 6574492 B, Ben-Haim
- US 20150351652 A, Marecki
- US 5391199 A
- US 6690963 B
- US 6484118 B
- US 6239724 B
- US 6618612 B
- US 6332089 B
- US 7756576 B
- US 7869865 B
- US 7848787 B
- WO 9605768 A
- US 20020065455 A1
- US 20030120150 A1
- US 20040068178 A1